# EUROPÄISCHE PATENTSCHRIFT

(11) **EP 2 802 612 B1**
(45) Veröffentlichungstag und Bekanntmachung des Hinweises auf die Patenterteilung: **06.04.2016**
(21) Anmeldenummer: 13700070.9
(22) Anmeldetag: 04.01.2013
(51) Int. Cl.: A61L 24/04, C08G 18/48, C08G 18/73, C09J 175/12

(54) **BETA-AMINOSÄUREESTER MODIFIZIERTE (ASPARTAT-)HÄRTER UND DESSEN VERWENDUNG IN POLYHARNSTOFF-GEWEBEKLEBSTOFFEN**
BETA AMINO ACID ESTER MODIFIED (ASPARTATE) HARDENERS AND USE OF SAME IN POLYURIC TISSUE ADHESIVES
DURCISSANT (À BASE D'ASPARTATE) MODIFIÉ PAR ESTER D'ACIDES BÊTA-AMINÉS ET SON UTILISATION DANS DES ADHÉSIFS TISSULAIRES EN POLYURÉE

(30) Priorität: 09.01.2012 EP 12150419
(43) Veröffentlichungstag der Anmeldung: 19.11.2014
(73) Patentinhaber: Medical Adhesive Revolution GmbH, 52074 Aachen (DE)
(72) Erfinder: EGGERT, Christoph, 50733 Köln (DE); HECKROTH, Heike, 51519 Odenthal (DE)
(74) Vertreter: Davepon, Björn
(86) Internationale Anmeldenummer: PCT/EP2013/050082
(87) Internationale Veröffentlichungsnummer: WO 2013/104563

(56) Entgegenhaltungen:
- EP-A1- 2 145 634
- EP-A1- 2 336 212

## Beschreibung

Die vorliegende Erfindung betrifft einen Beta-Aminosäureester, insbesondere ein Beta-Aminosäureester modifiziertes Aspartat, ein Verfahren zu dessen Herstellung sowie die Verwendung dieser Verbindung als Härter zur Herstellung von Polyurethanharnstoffen oder Polyharnstoffen, insbesondere für Gewebekleber.

Gewebekleber sind in unterschiedlichen Ausgestaltungen im Handel erhältlich. Hierzu gehören die Cyanacrylate Dermabond^{®} (Octyl-2-Cyanoacrylat) und Histoacryl Blue^{®} (Butyl-Cyanoacrylat). Voraussetzung für eine effiziente Klebung der Cyanacrylate sind allerdings trockene Untergründe. Bei starken Blutungen versagen derartige Klebstoffe.

Als Alternative zu den Cyanacrylaten stehen biologische Klebstoffe wie zum Beispiel BioGlue^{®}, eine Mischung aus Glutaraldehyd und Bovinem Serumalbumin, diverse kollagen- und gelatinebasierte Systeme (FloSeal^{®}) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal^{®} dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot^{®} oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der WO 2009/106245 A2 ist die Herstellung und Verwendung von Polyharnstoff-Systemen als Gewebekleber bekannt. Die hier offenbarten Systeme umfassen wenigstens zwei Komponenten. Dabei handelt es sich um einen aminofunktionellen Asparaginsäureester und ein isocyanatfunktionelles Präpolymer, das durch Umsetzung von aliphatischen Polyisocyanaten mit Polyesterpolyolen erhältlich ist. Die beschriebenen 2-Komponenten Polyharnstoff-Systeme können als Gewebekleber für den Verschluss von Wunden in menschlichen und tierischen Zellverbänden eingesetzt werden. Dabei kann ein sehr gutes Klebeergebnis erzielt werden.

EP 2 145 634 und EP 2 336 212 offenbaren medizinische Klebstoffe.

Um eine gute Mischbarkeit der beiden Komponenten des Polyharnstoff-Systems sicher zu stellen, sollte die Viskosität der Komponenten bei 23 °C möglichst kleiner als 10.000 mPas sein. Eine entsprechend niedrige Viskosität weisen Präpolymere mit NCO-Funktionalitäten von weniger als 3 auf. Wenn derartige Präpolymere eingesetzt werden, ist es notwendig, als zweite Komponente einen Asparaginsäureester mit einer Aminofunktionalität von mehr als 2 einzusetzen, da ansonsten kein polymeres Netzwerk hergestellt werden kann. Dies ist jedoch erforderlich, damit das Polyharnstoff-System bzw. eine daraus bestehende Klebenaht die gewünschten mechanischen Eigenschaften wie Elastizität und Festigkeit aufweist. Darüber hinaus ist bei der Verwendung difunktioneller Asparaginsäureester nachteilig, dass die Aushärtungszeit bis zu 24 h beträgt, wobei das Polyharnstoff-System selbst nach dieser Zeit in vielen Fällen klebrig bleibt, also nicht tack free ist. Ferner sind die hierbei entstehenden Klebstoffe in erster Linie für topische Anwendungen konzipiert und nicht innerhalb kurzer Zeit, beispielsweise innerhalb von 6 Monaten oder weniger im Körper biologisch abbaubar. Für eine Anwendung innerhalb des Körpers sollte ein Klebstoffsystem jedoch diese Voraussetzung erfüllen.

Aus der WO 2010/066356 sind Klebstoffsysteme für medizinische Anwendungen bekannt, in denen isocyanatterminierte Präpolymere mit sekundären Diaminen umgesetzt beziehungsweise ausgehärtet werden. Auch hier stellen sich die bereits in Bezug auf die WO 2009/106245 A2 genannten Nachteile ein.

Bei Gewebeklebstoffen ist also die Aushärtezeit neben der eigentlichen Klebkraft ein wichtiger Parameter. Wenn der Klebstoff zu schnell aushärtet, ist die offene Zeit, die dem Anwender zum Inkontaktbringen der zu verklebenden Wundränder verbleibt, möglicherweise zu gering. Umgekehrt ist eine zu lange Aushärtezeit unerwünscht, weil dadurch lange Wartezeiten entstehenden und die Wunde währenddessen fixiert werden muss, damit sich die zu verklebenden Wundränder nicht wieder voneinander separieren. Eine zweckmäßige Aushärtegeschwindigkeit kann beispielsweise von 1 bis 5 Minuten angegeben werden, wobei sich die optimale Aushärtezeit letztlich nach dem jeweiligen Anwendungszweck richtet. Während dieser Aushärtezeit sollte der Klebstoff dennoch möglichst lange verarbeitbar bleiben.

Es liegt auf der Hand, dass die Einstellung der gewünschten Aushärtezeit eine anspruchsvolle Aufgabe darstellt, denn hierfür muss der Härter auf das auszuhärtende Präpolymer beziehungsweise die Präpolymerzusammensetzung abgestimmt sein. Dabei kann für ein bestimmtes auszuhärtendes Präpolymer der Einsatz von Diaminen zu einer zu schnellen Aushärtung führen, andererseits die vorgenannten Aspartathärter zu langsam sein.

Vor diesem Hintergrund bestand die Aufgabe der Erfindung darin, eine Verbindung als neuen Härter zur Verfügung zu stellen, wobei diese Verbindung für verschiedene Polyurethanharnstoff-Systeme gewünschte Aushärtezeiten ermöglichen soll. Dabei sollte die Wirkung dieser Verbindung auf die Aushärtegeschwindigkeit möglichst in gewissen Bereichen einstellbar sein. Zudem ist die Gewährleistung einer ausreichenden biologischen Abbaubarkeit nach der Anwendung im tierischen oder menschlichen Körper wünschenswert.

Diese Aufgabe wird gelöst durch eine Verbindung der Formel (I) 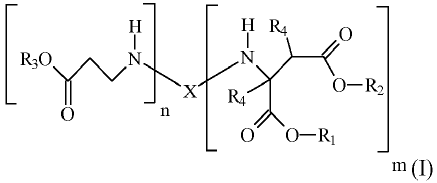 in der
- R₁, R₂, R₃: jeweils unabhängig voneinander gleiche oder verschiedene organische Reste, die keinen Zerewitinoff-aktiven Wasserstoff aufweisen sind,
- R₄: unabhängig voneinander Wasserstoff, gleiche oder verschiedene organische Reste sind, die keinen Zerewitinoff-aktiven Wasserstoff aufweisen, oder gemeinsam einen ungesättigten oder aromatischen Ring bilden, der gegebenenfalls Heteroatome enthalten kann, wobei R₄ keinen Zerewitinoff-aktiven Wasserstoff aufweist,
- X: ein linearer oder verzweigter, gegebenenfalls auch in der Kette mit Heteroatomen substituierter, organischer Rest ist, der keinen Zerewitinoff-aktiven Wasserstoff aufweist,
- n: 0 < n ≤ 2 und
- m: 0 ≤ m < 2,
wobei n + m = 2 ist.

Mit anderen Worten besitzt die vorgenannte Verbindung beta-Aminosäureestergruppen, sowie optional Aspartatestergruppen mit jeweils variablen Anteilen. Das heißt, n und m sind nicht zwangsweise ganzzahlig, sondern die beanspruchte Zusammensetzung kann eine Mischung verschieden substituierter Verbindungen darstellen, die unter die vorgenannte Formel (I) fallen. Hierbei kann die Mischung natürlich auch herstellungsbedingt einen Anteil an Diaspartaten enthalten, wobei dieser Anteil vorzugsweise weniger als 90 Mol-% bezogen auf die Gesamtstoffmenge der Verbindungen beträgt, insbesondere weniger als 75 Mol-%.

Überraschenderweise hat sich herausgestellt, dass Verbindungen dieses Typs eine hohe Aushärtegeschwindigkeit beim Einsatz als Härter in einem Polyurethanharnstoffsystem zeigen. Dabei kann die Aushärtegeschwindigkeit mittels Variation von n beziehungsweise m in bestimmten Bereichen an das gewünschte Maß angepasst werden. Die damit ausgehärteten Gewebekleber, beispielsweise auf Polyurethanharnstoffbasis, sind binnen kurzer Zeit tack free, was deren Anwendung erheblich vereinfacht.

In Ausgestaltung der erfindungsgemäßen Verbindung sind die Reste R₁, R₂, R₃ jeweils unabhängig voneinander lineare oder verzweigte, insbesondere gesättigte, aliphatische C1 bis C10 Kohlenwasserstoffreste, bevorzugt C2 bis C18, besonders bevorzugt C2 bis C6 und ganz besonders bevorzugt C2 bis C4.

Weiterhin kann der Rest X ein linearer, verzweigter oder zyklischer organischer C2 bis C16 Rest sein, bevorzugt C3 bis C14, besonders bevorzugt C4 bis C12. Hierbei stellt der Rest X insbesondere einen aliphatischen Kohlenwasserstoffrest dar. Besonders bevorzugte Reste sind ein 2-Methyl-pentamethylen-Rest, ein Hexamethylen-Rest oder ein Isophoryl-Rest, um nur einige Beispiele zu nennen. Grundsätzlich können auch Mischungen von Verbindungen mit unterschiedlichem X eingesetzt werden.

Um ein möglichst homogenes Aushärteverhalten zu ermöglichen können bei einer erfindungsgemäßen Verbindung die Reste R₁ und R₂ jeweils gleich sein, wobei insbesondere die Reste R₁, R₂ und R₃ gleich sein können.

Nach einer bevorzugten Ausführungsform der erfindungsgemäßen Verbindung gilt 0 < n < 2 und 0 < m < 2, wobei n insbesondere 0,5 bis 1,5 beträgt, vorzugsweise 0,6 bis 1,4, weiter bevorzugt 0,7 bis 1,3, besonders bevorzugt 0,8 bis 1,2 und ganz besonders bevorzugt 0,9 bis 1,1. Mit anderen Worten betrifft diese Ausgestaltung der Erfindung eine Mischung von Verbindungen der Formel (I), bei der statistisch zumindest ein Teil der Verbindungen sowohl eine beta-Aminosäuregruppe als auch eine Aspartatgruppe trägt. Im vorgenannten Zahlenbereich von n, m etwa gleich 1 umfasst diese Mischung statistisch betrachtet nahezu ausschließlich beta-Aminosäureester modifizierte Aspartate. Dies ist besonders vorteilhaft, weil durch Einstellen von n und m die Reaktivität des Härters variiert werden kann. So kann die Aushärtegeschwindigkeit beispielsweise eines Polyurethanharnstoffsystems dadurch erhöht werden, dass bei der erfindungsgemäßen Verbindung nach Formel (I) der Anteil an beta-Aminosäuregruppen, d.h. also die Laufziffer n, statistisch erhöht wird. Umgekehrt kann bei einer zu hohen Aushärtegeschwindigkeit der Anteil an Aspartatgruppen, also die Laufziffer m, erhöht werden.

Die zuvor geschilderte Anpassung des Anteils an beta-Aminosäuregruppen und Aspartatgruppen kann auf verschiedene Weisen bewerkstelligt werden. So kann durch Wahl eines entsprechenden Mischungsverhältnisses der Edukte zur Erzeugung der jeweiligen funktionellen Gruppen der Anteil dieser Gruppen bereits während der Herstellung eingestellt werden. Dies wird weiter unten nochmals erläutert. Denkbar ist ferner auch das Vermischen der reinen Di-beta-Aminosäureesterverbindung der Formel (I) (d.h. n= 2, m=0) bzw. der reinen Di-Aspartatverbindung der Formel (I) (d.h. n=0, m=2) mit der reinen beta-Aminosäureester modifizierten Aspartatverbindung (d.h. n, m= 1) der Formel (I) in einem entsprechenden Verhältnis. Wie bereits voranstehend erörtert wurde, kann diese Mischung auch einen Anteil an Di-Aspartaten und Di-beta-Aminosäureestern umfassen, wobei auch hier dieser Anteil vorzugsweise weniger als 90 Mol-% bezogen auf die Gesamtstoffmenge der Verbindungen beträgt, insbesondere weniger als 75 Mol-%.

Ein weiterer Gegenstand der vorliegenden Erfindung betrifft ein Verfahren zur Herstellung einer Verbindung nach einem der Ansprüche 1 bis 5, bei dem eine Diaminverbindung der allgemeinen Formel (II)

H₂N-X-NH₂ (II)

mit einem Acrylsäureester der allgemeinen Formel (III) 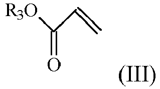 und gewünschtenfalls einem Dieester einer ungesättigten Dicarbonsäure der allgemeinen Formel (IV) 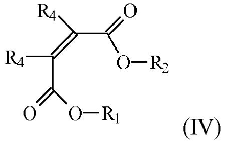 umgesetzt wird, wobei pro Mol der Diaminverbindung n Mol Acrylsäureester und m Mol Dieester eingesetzt werden,
und wobei
- R₁, R₂, R₃: jeweils unabhängig voneinander gleiche oder verschiedene organische Reste, die keinen Zerewitinoff-aktiven Wasserstoff aufweisen sind,
- R₄: unabhängig voneinander Wasserstoff, gleiche oder verschiedene organische Reste sind, die keinen Zerewitinoff-aktiven Wasserstoff aufweisen, oder einen ungesättigten oder aromatischen Ring bilden, der gegebenenfalls Heteroatome enthalten kann, wobei R₄ keinen Zerewitinoff-aktiven Wasserstoff aufweist,
- X: ein linearer oder verzweigter, gegebenenfalls auch in der Kette mit Heteroatomen substituierter, organischer Rest ist, der keinen Zerewitinoff-aktiven Wasserstoff aufweist,
- n: 0 < n ≤ 2,
- m: 0 ≤ m < 2
und n + m = 2 ist.

Bei dem erfindungsgemäßen Verfahren können prinzipiell sämtliche Arten von Diaminen eingesetzt werden. Diese weisen - abgesehen von den zwei primären Aminogruppen - keine Zerewitinoff-aktiven Wasserstoffatome auf.

Unter einem Zerewitinoff-aktiven H-Atom wird im Rahmen der vorliegenden Erfindung ein azides H-Atom oder "aktives" H-Atom verstanden. Ein solches kann in an sich bekannter Weise durch eine Reaktivität mit einem entsprechenden Grignard-Reagenz ermittelt werden. Die Menge an Zerewitinoff-aktiven H-Atomen wird typischerweise über die Methanfreisetzung gemessen, die bei einer Reaktion der zu überprüfenden Substanz mit Methylmagnesiumbromid (CH₃-MgBr) gemäß der folgenden Reaktionsgleichung (Formel 1) frei wird:

CH₃-MgBr + ROH → CH₄ + Mg (OR)Br (1)

Zerewitinoff-aktive H-Atome stammen typischerweise von C-H aziden organischen Gruppen, -OH, -SH, -NH₂ oder -NHR mit R als organischem Rest sowie -COOH.

Als Acrylsäureester kommen beispielsweise solche vom (Meth-)Acrylattyp in Frage. Hierbei kann beispielsweise auf C1 bis C12-Acrylate zurück gegriffen werden, insbesondere C1 bis C10-Acrylate, vorzugsweise C1 bis C8-Acrylate, weiter bevorzugt C2 bis C6-Acrylate.

Als Dieester einer ungesättigten Dicarbonsäure kommen beispielsweise Maleinsäureester oder die Ester der Tetrahydrophthalsäure in Betracht, insbesondere der 3,4,5,6-Tetrahydrophthalsäure sowie Kombinationen hiervon. Hierbei entsprechen beide Reste R₄ im Falle der Maleinsäureester jeweils einem Wasserstoffatom, wobei beide Reste R₄ im Falle der Tetrahydrophthalsäure gemeinsam einen ungesättigten 6er Ring bilden.

Unabhängig hiervon können die Diester aus C1 bis C12 Estern der jeweiligen Di-Säure ausgewählt sein, insbesondere aus den C1 bis C8 Estern, vorzugsweise aus den 2 bis C4 Estern.

Bei dem erfindungsgemäßen Verfahren werden pro Mol der Diaminverbindung n Mol Acrylsäureester und m Mol Dieester eingesetzt. Auf diese Weise lassen sich die vorstehend beschriebenen Mischungen der Verbindung nach Formel (I) unmittelbar herstellen. Damit kann die vorbeschriebene Anpassung der Aushärtegeschwindigkeit bereits durch eine entsprechende Steuerung des Herstellungsverfahrens vorgenommen werden.

Die Erfindung betrifft zudem eine Verbindung der Formel (I), die nach dem erfindungsgemäßen Verfahren herstellbar ist.

Ein weiterer Gegenstand der vorliegenden Erfindung betrifft ein Polyharnstoff System, das die folgenden Komponenten umfasst:
als Komponente A) isocyanatfunktionelle Präpolymere erhältlich durch Umsetzung von
aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) eine erfindungsgemäße Verbindung der allgemeinen Formel (I),
gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Präpolymeren gemäß Komponente A) mit Verbindungen gemäß Komponente B) und/oder organischen Füllstoffen gemäß Komponente C) und
gegebenenfalls als Komponente E) Wasser und/oder ein tertiäres Amin.

Die erfindungsgemäßen Polyharnstoff-Systeme werden durch Mischung der Präpolymere A) mit der erfindungsgemäßen Verbindung der allgemeinen Formel (I) B) sowie gegebenenfalls den Komponenten C), D) und/oder E) erhalten. Das Verhältnis von freien oder blockierten Aminogruppen zu freien NCO-Gruppen beträgt dabei bevorzugt 1:1,5, besonders bevorzugt 1:1. Wasser und/oder Amin werden dabei der Komponente B) bzw. C) beigemischt.

Die isocyanatfunktionellen Präpolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4`-isocyanatocyclohexyl)-methane oder deren Mischungen beliebigen Isomerengehalts, 1,4-Cyclohexylendüsocyanat, 4-Isocyanatomethyl-1,8-octandüsocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werden.

Ganz besonders bevorzugt wird Hexamethylendiisocyanat als Polyisocyanat A1) eingesetzt.

Gemäß einer bevorzugten Ausführungsform des erfindungsgemäßen Polyharnstoff-Systems ist vorgesehen, dass die Polyole A2) Polyesterpolyole und/oder Polyester-Polyether-Polyole und/oder Polyetherpolyole sind. Insbesondere bevorzugt sind dabei Polyester-Polyether-Polyole und/oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methylpentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligotetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylen-glykol, Polybutylenglykole, Bisphenol A und lactonmodifizierte Diole der vorstehend genannten Art in Frage.

Zur Herstellung des Präpolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Präpolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Präpolymere A) beträgt dabei bevorzugt 20 bis 120 °C und weiter bevorzugt 60 bis 100 °C.

Die hergestellten Präpolymere haben einen nach DIN EN ISO 11909 gemessenen mittleren NCO-Gehalt von 2 bis 10 Gew.-%, bevorzugt 2,5 bis 8 Gew.-%.

Gemäß einer weiteren Ausführungsform des erfindungsgemäßen Polyharnstoff-Systems können die Präpolymere A) eine mittlere NCO-Funktionalität von 2 bis 6, bevorzugt von 2,3 bis 4,5, weiter bevorzugt von 2,5 bis 4, ganz besonders bevorzugt von 2,7 bis 3,5 und insbesondere von 3 aufweisen.

Bei den organischen Füllstoffen der Komponente C) kann es sich bevorzugt um hydroxyfunktionelle Verbindungen, insbesondere um Polyetherpolyole mit sich wiederholenden Ethylenoxid Einheiten handelt.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Die Viskosität der organischen Füllstoffe gemessen nach DIN 53019 bei 23 °C beträgt bevorzugt 50 bis 4000 mPas, besonders bevorzugt 50 bis 2000 mPas.

In einer bevorzugten Ausführungsform des erfindungsgemäßen Polyharnstoff-Systems werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Um das mittlere Äquivalentgewicht der insgesamt zur Präpolymervernetzung eingesetzten Verbindungen bezogen auf die NCO-reaktiven Gruppen weiter zu reduzieren, ist es möglich zusätzlich Umsetzungsprodukte der Präpolymere A) mit der erfindungsgemäßen Verbindung der allgemeinen Formel (I) B) und/oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Equivalentgewicht und das Equivalentvolumen der Härterkomponente in größeren Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Gemäß einer weiteren bevorzugten Ausführungsform des erfindungsgemäßen Polyharnstoff-Systems ist vorgesehen, dass die Komponente E) ein tertiäre Amine der allgemeinen Formel (V) 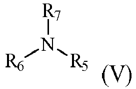 enthält, in der

R₅, R₆, R₇ unabhängig voneinander Alkyl- oder Heteroalkyreste mit Heteroatomen in der Alkylkette oder an deren Ende sein können, oder R₅ und R₆ gemeinsam mit dem sie tragenden Stickstoffatom einen aliphatischen, ungesättigten oder aromatischen Heterozyklus bilden können, der gegebenenfalls weitere Heteroatome enthalten kann.

Diese Polyharnstoff-Systeme zeichnen sich durch eine besonders schnelle Aushärtung aus.

Bei den in Komponente E) verwendeten Verbindungen kann es sich ganz besonders bevorzugt um tertiäre Amine ausgewählt aus der Gruppe Triethanolamin, Tetrakis (2-hydroxyethyl) ethylendiamin, N,N-Dimethyl-2-(4-methylpiperazin-l-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin) handeln.

Ganz besonders hohe Aushärtungsgeschwindigkeiten können auch erzielt werden, wenn die Komponente E) 0,2 bis 2,0 Gew.-% Wasser und/oder 0,1 bis 1,0 Gew.-% des tertiäres Amin enthält.

Selbstverständlich können in die Polyharnstoff-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe oder Kombinationen hiervon eingearbeitet werden.

Die Wirkstoffe können als reiner Wirkstoff oder aber in verkapselter Form vorliegen, um beispielsweise eine zeitlich verzögerte Abgabe zu erzielen. Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxid-freisetzende Komponente, bevorzugt L-Arginin oder eine L-Argininhaltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®), Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam®) geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF α; (Transforming Growth Factor alpha), TGF β (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen der erfindungsgemäßen Zusammensetzung.

Das erfindungsgemäße Polyharnstoff-System ist besonders zum Verschließen, Verbinden, Verkleben oder Abdecken von Zellgewebe und insbesondere zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschliches oder tierisches Zellgewebe eingesetzt werden. Mit seiner Hilfe können schnell aushärtende, stark am Gewebe anhaftende, transparente, flexible und biokompatible Klebnähte hergestellt werden.

Noch ein weiterer Gegenstand der Erfindung ist Dosiersystem mit zwei Kammern für ein erfindungsgemäßes Polyharnstoff-System, bei dem in der einen Kammer die Komponente A) und in der anderen Kammer die Komponenten B) und gegebenenfalls die Komponenten C), D) und. E) des Polyharnstoff-Systems enthalten sind. Ein derartiges Dosiersystem eignet sich insbesondere dafür das Polyharnstoff-System als Kleber auf Gewebe zu applizieren.

### Beispiele:

Die vorliegende Erfindung wird im Folgenden anhand von Ausführungsbeispielen näher erörtert.

### Methoden:

### Molekulargewicht:

Die Molekulargewichte wurden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 µl - 200 µl. Die Berechnung der Molmassenmittelwerte Mw; Mn und Mp sowie der Polydispersität Mw/Mn erfolgte softwaregestützt. Basislinienpunkte und Auswertegrenzen wurden entsprechend der DIN 55672 Teil 1 festgelegt.

### NCO-Gehalt:

Der NCO-Gehalt wurde, wenn nicht ausdrücklich anders erwähnt, volumetrisch gemäß DIN-EN ISO 11909 bestimmt.

### Viskosität:

Die Viskosität wurde nach ISO 3219 bei 23°C bestimmt.

### Restmonomergehalt:

Die Bestimmung des Restmonomergehalts erfolgte nach DIN ISO 17025.

Als NMR wurde ein Bruker DRX 700 Gerät verwendet.

### Synthese des NCO-terminierten Präpolymers A:

465 g HDI und 2.35 g Benzoylchlorid wurden in einem 1 1 Vierhalskolben vorgelegt. Innerhalb von 2 h wurden bei 80 °C 931.8 g eines trifunktionellen Polyethers (Produkt der Bayer MaterialScience AG) mit einem Ethylenoxidgehalt von 71 % und einem Propylenoxidgehalt von 29 %, jeweils bezogen auf dem gesamten Alkylenoxidgehalt, hinzugefügt und 1 h nachgerührt. Anschließend wurde durch Dünnschichtdestillation bei 130 °C und 0,13 mbar das überschüssige HDI abdestilliert. Man erhielt 980 g (71 %) des Präpolymers mit einem NCO-Gehalt von 2,53 % (Äquivalentgewicht: 1660 g/mol) und einer Viskosität von 4500 mPas/23°C. Der Restmonomerengehalt betrug < 0,03 % HDI.

### Synthese des Polyols B mit Lactid für das Präpolymer C:

In einem 2 Liter-Edelstahldruckreaktor werden 98,1 g eines auf Glycerin gestarteten Poly(oxypropylen)triols mit OH-Zahl = 400 mg KOH/g, 48,4 g Dilactid sowie 0,107 g DMC-Katalysator (hergestellt gemäß EP-A 700 949) unter Stickstoff vorgelegt und dann auf 100°C aufgeheizt. Nach 30 min Strippen mit Stickstoff bei 0,1 bar wird die Temperatur auf 130°C erhöht und bei dieser Temperatur dann eine Mischung aus 701,8 g Ethylenoxid und 217,8 g Propylenoxid innerhalb von 130 min dosiert. Nach einer Nachreaktionszeit von 45 min bei 130°C werden leichtflüchtige Anteile bei 90°C für 30 min im Vakuum abdestilliert und das Reaktionsgemisch anschließend auf Raumtemperatur abgekühlt.

Produkteigenschaften:

| | |
|---|---|
| OH-Zahl: | 33,7 mg KOH/g |
| Viskosität (25°C): | 1370 mPas |
| Polydispersität (Mw/Mn): | 1,13 |

### Synthese des NCO-terminierten Präpolymers C:

293 g HDI und 1,5 g Benzoylchlorid wurden in einem 1 1 Vierhalskolben vorgelegt. Innerhalb von 2h wurden bei 80°C 665,9 g Polyol B hinzugefügt und 1 h nachgerührt. Anschließend wurde durch Dünnschichtdestillation bei 130°C und 0,13 mbar das überschüssige HDI abdestilliert. Man erhielt das Präpolymer mit einem NCO-Gehalt von 2,37% (Äquivalentgewicht: 1772 g/mol). Der Restmonomerengehalt betrug < 0,03 % HDI. Viskosität: 5740 mPas/23°C

### Synthese der erfindungsgemäßen Härter:

Die erfindungsgemäßen Härter wurden jeweils ausgehend von einer Diaminverbindung Synthetisiert. Dabei wurden die folgenden Verbindungen hergestellt:

| Härter | Diamin / Acrylat | X | Äquvalentgewicht | Zeit bis zur Aushärtung mit Präpolymer A | Zeit bis zur Aushärtung mit Präpolymer C |
|---|---|---|---|---|---|
| HA1 | Dytek A / Ethylacrylat | 0,5 | 200,72 g/mol | 4 min | 5 min |
| HA2 | Dytek A / Ethylacrylat | 0,25 | 215,38 g/mol | 6 min | 8 min |
| HA3 | Dytek A / Ethylacrylat | 0,125 | 226,72 g/mol | 6,5 min | 8,5 min |
| HB1 | Hexamethylendiamin / Ethylacrylat | 0,5 | 208,95 g/mol | 3 min | 3 min |
| HB2 | Hexamethylendiamin / Ethylacrylat | 0,25 | 217,05 g/mol | 5 min | 5 min |
| HB3 | Hexamethylendiamin / Ethylacrylat | 0,125 | 221,34 g/mol | 5,5 min | 5,5 min |
| HCl | Isophoryldiamin / Ethylacrylat | 0,5 | 220,47 g/mol | 20 min | >5 h |
| HC2 | Isophoryldiamin / Ethylacrylat | 0,25 | 239,32 g/mol | > 5 h | >5 h |
| HC3 | Isophoryldiamin / Ethylacrylat | 0,125 | 247,79 g/mol | >5 h | >5 h |
| HD | Dytek A / Butylacrylat | 0,375 | 216,21 g/mol | 6 min | 7 min |
| HE | Hexamethylandiamin / Butylacrylat | 0,375 | 215,38 g/mol | 5 min | 5 min |
| HF | Isophoryldiamin / Butylacrylat | 0,375 | 239,32 g/mol | > 5 h | >5 h |

Zur Herstellung der vorgenannten Verbindungen wurde jeweils folgendermaßen vorgegangen:
0,5 mol des jeweiligen Diamins wurden bei RT vorgelegt (feste Amine wurden als Schmelze vorgelegt) und (1-x) mol Diethylmaleat (0 ≤ x ≤ 1) über 1 h zugetropft, so dass die Temperatur der Reaktionsmischung 60°C nicht überstieg. Nach 12 h Rühren bei RT wurden x mol Acrylat über 1 h zugetropft, so dass die Temperatur der Reaktionsmischung 60°C nicht überstieg. Nach abgeklungener Exothermie wurde das Reaktionsgemisch für 24 h bei 60°C gerührt.

Nach Abkühlung auf RT wurde zur Aufreinigung das Reaktionsgemisch in das dreifache Volumen an Wasser gegeben und konz. Salzsäure hinzugegeben, bis sich eine klare Lösung bildete (pH-Wert = 1). Die entstandene Lösung wurde dreimal mit dem gleichen Volumen an Ethylacetat oder Dichlormethan Extrahiert und die Phasen getrennt (organische Phasen werden verworfen). Die wässrige Phase wurde mit konzentrierter Natronlauge alkalisch gestellt (pH-Wert = 10) und mit gleichem Volumen Ethylacetat bzw. Dichlormethan abermals dreimal extrahiert und die Phasen getrennt. Die organische Phase wurde über Natriumsulfat getrocknet und das Lösungsmittel im Vakuum entfernt. Man erhielt gelbe Öle in quantitativen Ausbeuten.

### Aushärtungsversuche:

1 eq des Präpolymers A bzw. C wurden jeweils mit 1 eq des Härters (HA2-3, HB1-3, HC1-3, HD, HE, HF) in einem Plastikbecher vorgelegt und für 30 Sekunden gut miteinander vermischt. Anschließend wurde die Zeit gemessen bis die Mischung tack free war.

### In vitro Gewebeverklebungsversuch:

Zu 1 eq Präpolymer A wurden jeweils 1 eq eines Härters (HA2-3, HB1-3, HC1-3, HD, HE, HF) zugesetzt und in einem Becher 20 s sorgfältig verrührt. Das Polyharnstoff-System wurde unmittelbar danach auf das zu klebende Muskelgewebe als dünne Schicht aufgetragen. Als Verarbeitungszeit wurde dabei die Zeit bestimmt, innerhalb der das Klebstoff-System noch eine so niedrige Viskosität besaß, dass es problemlos auf das Gewebe aufgetragen werden konnte.

Die Zeit, nach der das Polyharnstoff-System nicht mehr klebrig war (tack free time) wurde durch Haftversuche mit einem Glasstab gemessen. Dazu wurde der Glasstab mit der Schicht aus dem Polyharnstoff-System in Kontakt gebracht. Blieb dieser nicht mehr haften, wurde die System als tack free angesehen. Zusätzlich wurde die Klebekraft bestimmt, indem zwei Stücke Muskelgewebe (l = 4 cm, h = 0.3 cm, b = 1 cm) an den Enden 1 cm weit mit dem Polyharnstoff-System bestrichen und überlappend geklebt wurden. Die Klebkraft des Polyharnstoff-Systems wurde jeweils durch Zug überprüft.

Die Ergebnisse der Gewebeklebeversuche mit dem Präpolymer A sind in der folgenden Tabelle zusammengefasst:

| Härter | Verarbeitungszeit | Tack free time | Klebekraft |
|---|---|---|---|
| HA1 | 0:30 min | 2:15 min | + |
| HA2 | 3:00 min | 3:10 min | ++ |
| HA3 | 3:30 min | 4:00 min | ++ |
| HB1 | 0:33 min | 1:50 min | + |
| HB2 | 2:00 min | 2:50 min | ++ |
| HB3 | 3:45 min | 3:15 min | ++ |

Die Ergebnisse belegen, dass insbesondere die Härter HA2, HA3, HB2 und HB3 eine vergleichsweise lange Verarbeitungszeit bei dennoch kurzer tack free Zeit sowie guter Klebkraft miteinander vereinen. Demgegenüber sind die Härter HA1 und HB1 besonders schnell tack free und entsprechend weniger lange verarbeitbar. Zudem zeichnen sich diese Härter durch eine geringfügig verminderte Klebkraft im Vergleich zu den übrigen Härtern aus.

## Patentansprüche

1. Verbindung der Formel (I) 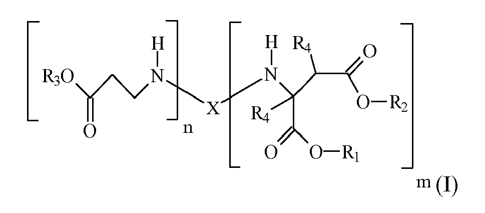 in der
R₁, R₂, R₃ jeweils unabhängig voneinander gleiche oder verschiedene organische Reste, die keinen Zerewitinoff-aktiven Wasserstoff aufweisen sind,
R₄ unabhängig voneinander Wasserstoff, gleiche oder verschiedene organische Reste sind, die keinen Zerewitinoff-aktiven Wasserstoff aufweisen, oder gemeinsam einen ungesättigten oder aromatischen Ring bilden, der gegebenenfalls Heteroatome enthalten kann, wobei R₄ keinen Zerewitinoff-aktiven Wasserstoff aufweist,
X ein linearer oder verzweigter, gegebenenfalls auch in der Kette mit Heteroatomen substituierter, organischer Rest ist, der keinen Zerewitinoff-aktiven Wasserstoff aufweist,
n 0 < n ≤ 2 und
m 0 ≤ m < 2,
wobei n + m = 2 ist.

2. Verbindung nach Anspruch 1, **dadurch gekennzeichnet, dass** die Reste R₁, R₂, R₃ jeweils unabhängig voneinander lineare oder verzweigte, insbesondere gesättigte, aliphatische C1 bis C10, bevorzugt C2 bis C18, besonders bevorzugt C2 bis C6 und ganz besonders bevorzugt C2 bis C4 Kohlenwasserstoffreste sind.

3. Verbindung nach einem der Ansprüche 1 oder 2, **dadurch gekennzeichnet, dass** der Rest X ein linearer, verzweigter oder zyklischer organischer C2 bis C16 Rest ist, bevorzugt C3 bis C14, besonders bevorzugt C4 bis C12 und insbesondere ein aliphatischer Kohlenwasserstoffrest ist.

4. Verbindung nach einem der vorstehenden Ansprüche, **dadurch gekennzeichnet, dass** jeweils die Reste R₁ und R₂ gleich sind, wobei insbesondere die Reste R₁, R₂ und R₃ gleich sind.

5. Verbindung nach einem der vorstehenden Ansprüche, **dadurch gekennzeichnet, dass** 0 < n < 2 und 0 < m < 2 sind, wobei n insbesondere 0,5 bis 1,5 beträgt, vorzugsweise 0,6 bis 1,4, weiter bevorzugt 0,7 bis 1,3, besonders bevorzugt 0,8 bis 1,2 und ganz besonders bevorzugt 0,9 bis 1,1.

6. Verfahren zur Herstellung einer Verbindung nach einem der Ansprüche 1 bis 5, bei dem eine Diaminverbindung der allgemeinen Formel (II)
H₂N-X-NH₂ (II)
mit einem Acrylsäureester der allgemeinen Formel (III) 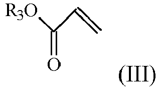 und gewünschtenfalls einem Dieester einer ungesättigten Dicarbonsäure der allgemeinen Formel (IV) 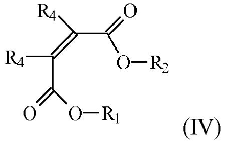 umgesetzt wird, wobei pro Mol der Diaminverbindung n Mol Acrylsäureester und m Mol Dieester eingesetzt werden,
und wobei
R₁, R₂, R₃ jeweils unabhängig voneinander gleiche oder verschiedene organische Reste, die keinen Zerewitinoff-aktiven Wasserstoff aufweisen sind,
R₄ unabhängig voneinander Wasserstoff, gleiche oder verschiedene organische Reste sind, die keinen Zerewitinoff-aktiven Wasserstoff aufweisen, oder einen ungesättigten oder aromatischen Ring bilden, der gegebenenfalls Heteroatome enthalten kann, wobei R₄ keinen Zerewitinoff-aktiven Wasserstoff aufweist,
X ein linearer oder verzweigter, gegebenenfalls auch in der Kette mit Heteroatomen substituierter, organischer Rest ist, der keinen Zerewitinoff-aktiven Wasserstoff aufweist,
n 0 < n ≤ 2,
m 0≤m<2
und n + m = 2 ist.

7. Polyharnstoff-System, umfassend
als Komponente A) isocyanatfunktionelle Präpolymere erhältlich durch Umsetzung von
aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) Verbindung gemäß einem der Ansprüche 1 bis 5, gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Präpolymeren gemäß Komponente A) mit Verbindungen gemäß Komponente B) und/oder organischen Füllstoffen gemäß Komponente C) und
gegebenenfalls als Komponente E) Wasser und/oder ein tertiäres Amin.

8. Polyharnstoff-System nach Anspruch 7, **dadurch gekennzeichnet, dass** die Polyole A2) Polyesterpolyole und/oder Polyester-Polyether-Polyole und/oder Polyetherpolyole, insbesondere Polyester-Polyether-Polyole und/oder Polyetherpolyole mit einem Ethylenoxidanteil zwischen 60 und 90 Gew.-%, enthalten.

9. Polyharnstoff-System nach einem der Ansprüche 7 oder 8, **dadurch gekennzeichnet, dass** es sich bei den organischen Füllstoffen der Komponente C) um hydroxyfunktionelle Verbindungen, insbesondere um Polyetherpolyole mit sich wiederholenden Ethylenoxid Einheiten handelt.

10. Polyharnstoff-System nach einem der Ansprüche 7 bis 9, **dadurch gekennzeichnet, dass** die Komponente E) ein tertiäres Amin der allgemeinen Formel (V) 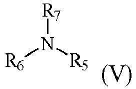 enthält, in der
R₅, R₆, R₇ unabhängig voneinander Alkyl- oder Heteroalkyreste mit Heteroatomen in der Alkylkette oder an deren Ende sein können, oder R₅ und R₆ gemeinsam mit dem sie tragenden Stickstoffatom einen aliphatischen, ungesättigten oder aromatischen Heterozyklus bilden können, der gegebenenfalls weitere Heteroatome enthalten kann.

11. Polyharnstoff-System nach einem der Ansprüche 7 bis 10, **dadurch gekennzeichnet, dass** das tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethyl-amino)ethyl](methyl)amino}ethanol, 3,3',3"-(1,3,5-Triazinan-1,3,5-triyl)tris(N,N-dimethyl-propan-1-amin) ist.

12. Polyharnstoff-System nach einem der Ansprüche 7 bis 11, **dadurch gekennzeichnet, dass** Komponente E) 0,2 bis 2,0 Gew.-% Wasser und/oder 0,1 bis 1,0 Gew.-% des tertiäres Amin enthält.

13. Polyharnstoff-System nach einem der Ansprüche 7 bis 12 zum Verschließen, Verbinden, Verkleben oder Abdecken von Zellgewebe, insbesondere zur Stillung des Austritts von Blut oder Gewebeflüssigkeiten oder dem Verschluss von Leckagen in Zellgewebe.

14. Polyharnstoff-System nach Anspruch 13 zur Verwendung zum Verschließen, Verbinden, Verkleben oder Abdecken von menschliches oder tierisches Zellgewebe.

15. Dosiersystem mit zwei Kammern für ein Polyharnstoff-System nach einem der Ansprüche 7 bis 14, **dadurch gekennzeichnet, dass** in der einen Kammer die Komponente A) und in der anderen Kammer die Komponenten B) und gegebenenfalls die Komponenten C), D) und. E) des Polyharnstoff-Systems enthalten sind.

## Claims

1. Compound of a formula (I) 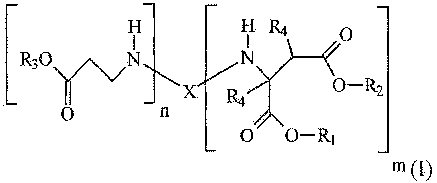 in which
R₁, R₂, R₃ are respectively independent equal or various organic radicals that have no Zerewitinoff active hydrogen,
R₄ are independently hydrogen, equal or different organic radicals, having no Zerewitinoff active hydrogen, or together form an unsaturated or aromatic ring, which can potentially contain heteroatoms, wherein R₄ have no Zerewitinoff active hydrogen,
X is a linear or branched, potentially even a substituted organic radical in the chain with heteroatom, which does not have Zerewitinoff active hydrogen,
n 0 < n ≤ 2 and
m 0 ≤ m ≤ 2,
wherein n + m = 2.

2. Compound according to Claim 1, wherein said radicals R₁, R₂, R₃ are respectively independently linear or branched, particularly saturated, aliphatic C1 to C10, preferably C2 to C18, particularly preferably C2 to C6, and very particularly preferably C2 to C4 hydrocarbon radicals.

3. Compound according to one of the Claims 1 or 2, wherein a radical X is linear, branched or cyclical, organic C2 to C16 radical, preferably C3 to C14, particularly preferably C4 to C12, and particularly an aliphatic hydrocarbon radical.

4. Compound according to one of the previous Claims, wherein said radicals R₁ and R₂ are equal, wherein particularly said radicals R₁, R₂, and R₃ are equal.

5. Compound according to one of the previous Claims, wherein 0 < n < 2 and 0 < m < 2, wherein n is particularly 0.5 to 1.5, preferably 0.6 to 1.4, more preferably 0.7 to 1.3, particularly preferably 0.8 to 1.2, and very particularly preferably 0.9 to 1.1.

6. Process for producing a compound according to one of the Claims 1 to 5, for which a diamine compound of a general formula (II)
H₂N-X-NH₂ (II)
is reacted with an acrylic acid ester of a general formula (III) 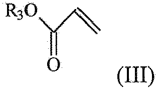 and, if desired, with a diester of an unsaturated dicarboxylic acid of a general formula (IV) 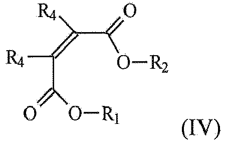 , wherein n mol of acrylic acid ester and m mol of diester is used per mol of diamine compound,
and wherein
R₁, R₂, R₃ are respectively independent equal or various organic radicals that have no Zerewitinoff active hydrogen,
R₄ are independently hydrogen, equal or different organic radicals, having no Zerewitinoff active hydrogen, or form an unsaturated or aromatic ring, which can potentially contain heteroatoms, wherein R₄ have no Zerewitinoff active hydrogen,
X is a linear or branched, potentially even a substituted organic radical in the chain with heteroatom, which does not have Zerewitinoff active hydrogen,
n 0 < n ≤ 2,
m 0≤m<2
and n + m = 2.

7. Polyurea system comprising
isocyanate functional prepolymers as a component A) can be achieved through a reaction of
aliphatic polyisocyanates A1) with
polyols A2), which may particularly have a number average molecular weight of ≥ 400 g/mol and an average OH functionality of 2 to 6,
a compound according to one of the Claims 1 to 5 as a component B),
potentially organic fillers, which may in particular have a viscosity measured according to DIN 53019 at 23 °C in the range of 10 to 6000 mPa, as a component C),
reaction products of isocyanate functional prepolymers according to component A) having compounds according to component B) and/or organic filler according to component C) potentially as component D), and
potentially water and/or a tertiary amine as a component E).

8. Polyurea system according to Claim 7, wherein said polyols A2) contain polyester polyols and/or polyester-polyether polyols and/or polyether polyols, particularly polyester-polyether polyols and/or polyether polyols having an ethylene oxide share of between 60 and 90% by weight.

9. Polyurea system according to one of the Claims 7 or 8, wherein said organic fillers of a component C) are hydroxy functional compounds, particularly polyether polyols having repeating ethylene oxide units.

10. Polyurea system according to one of the Claims 7 to 9, wherein a component E) contains a tertiary amine of a general formula (V) 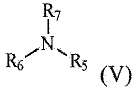 , in which
R₅, R₆, R₇ may independently be alkyl or heteroalkyl radicals having heteroatoms in an alkyl chain or at their ends, or R₅ and R₆ can form an aliphatic, unsaturated or aromatic heterocycle together with the nitrogen atom bearing them, which can potentially contain additional heteroatoms.

11. Polyurea system according to one of the Claims 7 to 10, wherein said tertiary amine is selected from a group of triethanolamine, tetrakis (2-hydroxyethyl) ethylenediamine, N,N-dimethyl-2-(4-methylpiperazine-1-yl)ethanamine, 2-{[2-(dimethylamino)ethyl](methyl)amino} ethanol, 3,3',3"-(1,3,5-Triazinan-1,3,5-triyl)tris(N,N-dimethyl-propane-1-amine).

12. Polyurea system according to one of the Claims 7 to 11, wherein said component E) contains 0.2 to 2.0% by weight of water and/or 0.1 to 1.0% by weight of tertiary amine.

13. Polyurea system according to one of the Claims 7 to 12 for closing, bonding, adhering or covering cell tissue, particularly for stopping the discharge of blood or tissue fluids or for closing leakages in cell tissue.

14. Polyurea system according to Claim 13 for the use of closing, bonding, adhering or covering human or animal cell tissue.

15. Dispensing system having two chambers for a polyurea/polyurethane system according to one of the Claims 7 to 14, wherein said component A) is contained in one chamber and said components B) and potentially said components C), D), and in another. E) of said polyurea system.

## Revendications

1. Composé de formule (I) 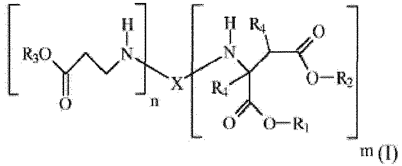 où
R₁, R₂, R₃ sont chacun, indépendamment l'un de l'autre, des groupes fonctionnels organiques identiques ou différents ne présentant aucun atome d'hydrogène actif de Zerewitinoff,
R4 sont, indépendamment l'un de l'autre, des groupes hydrogène ou des groupes fonctionnels organiques identiques ou différents ne présentant aucun atome d'hydrogène actif de Zerewitinoff, ou forment conjointement un cycle insaturé ou aromatique qui peut, le cas échéant, contenir des hétéroatomes, R4 ne présentant aucun atome d'hydrogène actif de Zerewitinoff,
X est un groupe fonctionnel organique linéaire ou ramifié, le cas échéant substitué dans la chaîne par des hétéroatomes, ne présentant aucun atome d'hydrogène actif de Zerewitinoff,
n 0 < n ≤ 2 et
m 0 ≤ m < 2,
où n + m = 2.

2. Composé selon la revendication 1, **caractérisé en ce que** les groupes fonctionnels R₁, R₂, R₃ sont chacun, indépendamment l'un de l'autre, des groupes fonctionnels hydrocarbures linéaires ou ramifiés, en particulier saturés, aliphatiques en C1 à C10, de préférence en C2 à C18, plus préférablement en C2 à C6, de façon tout particulièrement préférée en C2 à C4.

3. Composé selon l'une des revendications 1 ou 2, **caractérisé en ce que** le groupe fonctionnel X est un groupe fonctionnel organique linéaire, ramifié ou cyclique en C2 à C16, de préférence en C3 à C14, plus préférablement en C4 à C12 et particulièrement un groupe fonctionnel hydrocarbure aliphatique.

4. Composé selon l'une des revendications précédentes, **caractérisé en ce que** les groupes fonctionnels R₁ et R₂ sont chacun identiques, les groupes fonctionnels R₁, R₂ et R₃ étant particulièrement identiques.

5. Composé selon l'une des revendications précédentes, **caractérisé en ce que** 0 < n < 2 et 0 < m < 2, n valant notamment 0,5 à 1,5, de préférence 0,6 à 1,4, plus préférablement 0,7 à 1,3, particulièrement préférablement 0,8 à 1,2 et tout particulièrement préférablement 0,9 à 1,1.

6. Procédé de fabrication d'un composé selon l'une des revendications 1 à 5, par lequel un composé diamine de formule générale (II)
H₂N-X-NH₂ (II)
est mis en réaction avec un ester d'acide acrylique de formule générale (III) 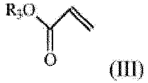 et, éventuellement, un diester d'un acide dicarboxylique insaturé de formule générale (IV) 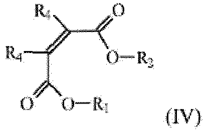 où n mole(s) d'ester d'acide acrylique et m mole(s) de diester par mole de composé diamine sont réagies,
et ou
R₁, R₂, R₃ sont chacun, indépendamment l'un de l'autre, des groupes fonctionnels organiques identiques ou différents ne présentant aucun atome d'hydrogène actif de Zerewitinoff,
R₄ sont, indépendamment l'un de l'autre, des groupes hydrogène ou des groupes fonctionnels organiques identiques ou différents ne présentant aucun atome d'hydrogène actif de Zerewitinoff, ou forment un cycle insaturé ou aromatique qui peut, le cas échéant, contenir des hétéroatomes, R₄ ne présentant aucun atome d'hydrogène actif de Zerewitinoff,
X est un groupe fonctionnel organique linéaire ou ramifié, le cas échéant substitué dans la chaîne par des hétéroatomes, ne présentant aucun atome d'hydrogène actif de Zerewitinoff,
n 0 < n ≤ 2,
m 0 ≤ m < 2
et n + m = 2.

7. Système de polyurée comprenant
comme composant A) des prépolymères à fonctionnalité isocyanate pouvant être obtenus par mise en réaction de polyisocyanates aliphatiques A1) avec des polyols A2), lesquels peuvent présenter en particulier un poids moléculaire moyen en nombre supérieur ou égal à 400 g/mol et une fonctionnalité OH moyenne de 2 à 6,
comme composant B) un composé selon l'une des revendications 1 à 5, éventuellement, comme composant C) des matières de charge organiques pouvant notamment présenter une viscosité mesurée à 23 °C selon DIN 53019 comprise entre 10 et 6000 mPas,
éventuellement, comme composant D) des produits de réaction de prépolymères à fonctionnalité isocyanate selon le composant A) avec des composés selon le composant B) et/ou des matières de charge organiques selon le composant C) et
éventuellement, comme composant E) de l'eau et/ou une amine tertiaire.

8. Système de polyurée selon la revendication 7, **caractérisé en ce que** les polyols A2) comportent des polyesterpolyols et/ou des polyester-polyéther-polyols et/ou des polyétherpolyols, en particulier des polyester-polyéther-polyols et/ou des polyétherpolyols ayant une part en oxyde d'éthylène comprise entre 60 et 90 % en poids.

9. Système de polyurée selon l'une des revendications 7 ou 8, **caractérisé en ce que** les matières de charge organiques du composant C) sont des composés hydroxyfonctionnels, en particulier des polyétherpolyols ayant des unités d'oxyde d'éthylène se répétant.

10. Système de polyurée selon l'une des revendications 7 à 9, **caractérisé en ce que** le composant E) contient une amine tertiaire de formule générale (V) 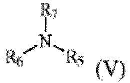 où
R₅, R₆, R₇ peuvent être, indépendamment l'un de l'autre, des groupes fonctionnels alkyle ou hétéroalkyle présentant des hétéroatomes dans la chaîne alkyle ou sur leur extrémité, ou R₅ et R₆ peuvent former un hétérocycle aliphatique, insaturé ou aromatique conjointement avec l'atome d'azote les portant, lequel peut éventuellement contenir d'autres hétéroatomes.

11. Système de polyurée selon l'une des revendications 7 à 10, **caractérisé en ce que** l'amine tertiaire est sélectionnée dans le groupe triéthanolamine, tetrakis (2-hydroxyéthyl)éthylènediamine, N,N-diméthyl-2-(4-méthylpipérazin-1-yl)éthanamine, 2-{[2-(diméthylamino)éthyl](méthyl)amino}éthanol, 3,3',3"-(1,3,5-triazinan-1,3,5-triyl)tris(N,N-diméthyl-propan-1-amine).

12. Système de polyurée selon l'une des revendications 7 à 11, **caractérisé en ce que** le composant E) contient 0,2 à 2,0 % en poids d'eau et/ou 0,1 à 1,0 % en poids d'amine tertiaire.

13. Système de polyurée selon l'une des revendications 7 à 12 destiné à fermer, lier, agglutiner ou recouvrir des tissus cellulaires, notamment dans le but de stopper l'écoulement de sang ou de fluides tissulaires, ou de sceller des fuites dans les tissus cellulaires.

14. Système de polyurée selon la revendication 13 destiné à être utilisé pour fermer, lier, agglutiner ou recouvrir des tissus cellulaires humains ou animaux.

15. Système de dosage à deux chambres pour un système de polyurée selon l'une des revendications 7 à 14, **caractérisé en ce que** le composant A) est contenu dans ladite première chambre et les composants B) sont contenus dans ladite seconde chambre, ainsi que, le cas échéant, les composants C), D) et E) du système de polyurée.
